# EUROPÄISCHE PATENTANMELDUNG

(11) **EP 1 655 008 A2**
(43) Veröffentlichungstag der Anmeldung: **10.05.2006**
(21) Anmeldenummer: 05110298.6
(22) Anmeldetag: 03.11.2005
(51) Int. Cl.: A61F 17/00

(54) **Verbandstoffbehälter**

(30) Priorität: 05.11.2004 DE 102004053524
(71) Anmelder: wero-medical Werner Michallik GmbH & Co. KG, 65232 Taunusstein (DE)
(72) Erfinder: MICHALLIK, Wolfram, 65187, Wiesbaden (DE)
(74) Vertreter: Weber, Dieter

(57) **Zusammenfassung**

Die vorliegende Erfindung betrifft einen Verbandstoffbehälter mit einem ersten Behälter (1), vorzugsweise für Verbandmaterial, und einem zweiten Behälter (2). Um einen Verbandstoffbehälter bereitzustellen, bei dem Verbandstoffe, insbesondere Pflaster, schnell und einfach zugänglich und entnehmbar sind, und der darüber hinaus Raum für das Unterbringen von weiterem Material bietet, wie beispielsweise einem Meldeberichtsheft, Schreibwerkzeug oder einer Flasche mit Desinfektionslösung, wird erfindungsgemäß vorgeschlagen, daß der erste Behälter (1) eine Vorderwand (10), eine Rückwand, Seitenwände (12, 13), eine in vertikaler Richtung nach oben offene Behälteröffnung (14) und einen Klappdeckel (15) zum Verschließen des Behälters (1) aufweist, der zweite Behälter (2) eine Rückwand, Seitenwände (22), eine Oberwand (25), eine Bodenwand und eine gegenüber der Rückwand in horizontaler Richtung nach vorne offene Behälteröffnung aufweist, der erste Behälter (1) an oder in der Nähe der oberen Kante der Rückwand des ersten Behälters (1) über ein Scharnier (27) an oder in der Nähe der in horizontaler Richtung vorderen Kante der Oberwand (25) des zweiten Behälters (2) klappbar angelenkt ist, der Klappdeckel (15) an oder in der Nähe der in horizontaler Richtung vorderen Kante der Oberwand (25) des zweiten Behälters (2) oder an oder in der Nähe der oberen Kante der Rückwand des ersten Behälters (1) über ein Scharnier (16) aufklappbar angelenkt ist und sich im geschlossenen Zustand über die Öffnung (14) bis an die obere Kante der Vorderwand (10) oder über diese hinaus erstreckt, die Anordnung und Bemessung der Behälterwände so ist, daß die Rückwand des ersten Behälters (1) die Behälteröffnung des zweiten Behälters (2) im wesentlichen vollständig verschließt bzw. abdeckt, wenn sich der erste Behälter im heruntergeklappten Zustand befindet.

## Beschreibung

Die vorliegende Erfindung betrifft einen Verbandstoffbehälter.

Verbandstoffe gehören zur Grundausstattung beim Erste-Hilfe-Material im Haushalt wie auch in Gewerbebetrieben. Solche Verbandstoffe umfassen unter anderem Pflaster, Binden, Kompressen usw. Des weiteren kann das Erste-Hilfe-Material beispielsweise eine Schere zum Zuschneiden von Binden oder Kompressen, Desinfektionslösungen und weitere Hilfsmittel für das Anlegen und Fixieren von Verbänden beinhalten. In Gewerbebetrieben ist es in der Regel vorgeschrieben, nach einer Verletzung bzw. einem Unfall einen Unfallmeldebericht zu erstellen. Hierfür steht häufig ein Berichtsheft zur Verfügung, in welches die Verletzung bzw. der Unfall mit Datum, Uhrzeit und Hergang einzutragen ist. Steht ein solches Berichtsheft und/oder Schreibwerkzeug nicht ortsnah zum Erste-Hilfe-Material zur Verfügung, so besteht gerade bei kleineren, weniger schweren Verletzungen, die schnell und einfach mit einem Pflaster oder ähnlichem verbunden werden können, die Neigung, das Erfordernis, einen Unfallmeldebericht zu erstellen, zu übergehen. Dies könnte dadurch ausgeräumt werden, wenn das Meldeberichtsheft und entsprechendes Schreibwerkzeug in unmittelbarer Nähe des Erste-Hilfe- Materials untergebracht und leicht zugänglich wären. In größeren Erste-Hilfe-Kästen, die üblicherweise in der Nähe des Arbeitsbereiches an der Wand montiert sind, werden Meldeberichtsheft und Schreibwerkzeug in der Regel einfach in den Erste-Hilfe-Kasten eingelegt, da hierfür ausreichend Raum vorhanden ist. Im Arbeitsbereich werden jedoch für geringfügigere Verletzungen üblicherweise auch kleinere Verbandstoffbehälter, wie Pflasterspender, montiert. Auch bei geringfügigen Verletzungen ist ein Unfallmeldebericht zu erstellen, jedoch bieten solche kleineren Verbandstoffbehälter bekannter Art keinen Raum für das Unterbringen von Meldeberichtsheft und Schreibwerkzeug. Darüber hinaus kann es erwünscht sein, bei z. B. einem Pflasterspender auch gleichzeitig eine Flasche mit Desinfektionslösung oder ähnlichem bereitzustellen. Auch hierfür bieten viele derzeit verwendete kleinere Verbandstoffbehälter, wie Pflasterspender, keinen Raum.

Die Aufgabe der vorliegenden Erfindung bestand daher darin, die vorgenannten Nachteile zu überwinden und einen Verbandstoffbehälter bereitzustellen, bei dem Verbandstoffe, insbesondere Pflaster, schnell und einfach zugänglich und entnehmbar sind, und der darüber hinaus Raum für das Unterbringen von weiterem Material bietet, wie beispielsweise einem Meldeberichtsheft, Schreibwerkzeug oder einer Flasche mit Desinfektionslösung.

Gelöst wird diese Aufgabe erfindungsgemäß durch einen Verbandstoffbehälter mit einem ersten Behälter, vorzugsweise für Verbandmaterial, und einem zweiten Behälter, wobei der erste Behälter eine Vorderwand, eine Rückwand, Seitenwände, eine in vertikaler Richtung nach oben offene Behälteröffnung und einen Klappdeckel zum Verschließen des Behälters aufweist, der zweite Behälter eine Rückwand, Seitenwände, eine Oberwand, eine Bodenwand und eine gegenüber der Rückwand in horizontaler Richtung nach vorne offene Behälteröffnung aufweist, der erste Behälter an oder in der Nähe der oberen Kante der Rückwand des ersten Behälters über ein Scharnier an oder in der Nähe der in horizontaler Richtung vorderen Kante der Oberwand des zweiten Behälters klappbar angelenkt ist, der Klappdeckel an oder in der Nähe der in horizontaler Richtung vorderen Kante der Oberwand des zweiten Behälters oder an oder in der Nähe der oberen Kante der Rückwand des ersten Behälters über ein Scharnier aufklappbar angelenkt ist und sich im geschlossenen Zustand über die Öffnung bis an die obere Kante der Vorderwand oder über diese hinaus erstreckt, die Anordnung und Bemessung der Behälterwände so ist, daß die Rückwand des ersten Behälters die Behälteröffnung des zweiten Behälters im wesentlichen vollständig verschließt bzw. abdeckt, wenn sich der erste Behälter im heruntergeklappten Zustand befindet.

Üblicherweise ist ein solcher erfindungsgemäßer Verbandstoffbehälter für die Montage an einer Wand vorgesehen. Der erste Behälter, der dabei von der Wand aus vor dem zweiten Behälter angeordnet ist, enthält zweckmäßigerweise Verbandmaterial und ist ganz besonders bevorzugt als Pflasterspender ausgestaltet. Der erste Behälter ist somit schnell und einfach zugänglich, damit ein Verletzter zunächst auf schnelle und einfache Weise das Verbandmaterial entnehmen und die Verletzung verbinden kann. Der an dem ersten Behälter vorgesehene Klappdeckel verhindert weitestgehend das Eindringen von Schmutz in den ersten Behälter und damit die Verunreinigung des Verbandmaterials.

Der hinter dem ersten Behälter angeordnete zweite Behälter weist eine Öffnung in Richtung des ersten Behälters und damit dem Benutzer zugewandt auf. Der erste Behälter ist an dem zweiten Behälter klappbar angelenkt befestigt, so daß er vom Benutzer für den Zugang zum Inneren des zweiten Behälters nach oben geklappt werden kann. Die Rückwand des ersten Behälters stellt somit den Verschluß bzw. die Abdeckung der Öffnung des zweiten Behälters dar.

Der zweite Behälter ist für das Unterbringen von weiterem Material zusätzlich zu dem Verbandmaterial im ersten Behälter vorgesehen, wie beispielsweise ein Meldeberichtsheft, einen Stift und/oder eine Flasche mit Desinfektionslösung.

Bei einer besonders bevorzugten Ausführungsform des erfindungsgemäßen Verbandstoffbehälters ist die Tiefe der Oberwand des zweiten Behälters von der Vorderkante bis zur Hinterkante im Verhältnis zur Tiefe des gesamten ersten Behälters wenigstens so groß, daß die Rückwand des ersten Behälters im hochgeklappten Zustand in Richtung der Rückwand des zweiten Behälters hinter der Senkrechten auf das Scharnier zu liegen kommt und sich die Abmessungen des ersten Behälters dabei nicht weiter als bis zu der durch die Rückwand des zweiten Behälters definierten vertikalen Ebene erstrecken.

Hierdurch wird gewährleistet, daß der erste Behälter, wenn er zum Freigeben der Öffnung des zweiten Behälters hochgeklappt wird, in dem hochgeklappten Zustand verbleibt und nicht vom Benutzer in diesem hochgeklappten Zustand gehalten werden muß. Dies ermöglicht dem Benutzer, den erfindungsgemäßen Verbandstoffbehälter mit nur einer Hand zu bedienen und Material aus dem Inneren des zweiten Behälters zu entnehmen, was im Falle von beispielsweise einer Handverletzung besonders vorteilhaft ist.

Bei einer weiteren bevorzugten Ausführungsform des erfindungsgemäßen Verbandstoffbehälters ist der Klappdeckel an oder in der Nähe der in horizontaler Richtung vorderen Kante der Oberwand des zweiten Behälters über ein Scharnier aufklappbar angelenkt. Dabei weisen besonders bevorzugt das Scharnier, über welches der Klappdeckel an dem zweiten Behälter angelenkt ist, und das Scharnier, über welches der erste Behälter an dem zweiten Behälter angelenkt ist, eine gemeinsame Scharnierachse auf. Die Scharnierhülsen, die über Haltestege jeweils mit dem ersten Behälter, dem zweiten Behälter bzw. mit dem Klappdeckel verbunden sind, liegen bei dieser Anordnung vorteilhafterweise unmittelbar nebeneinander und sind um die gemeinsame Scharnierachse drehbar bzw. schwenkbar gelagert.

Wie oben bereits ausgeführt wurde, ist eine besonders vorteilhafte Anwendung des erfindungsgemäßen Verbandstoffbehälters das Unterbringen von Verbandmaterial für eher geringfügige Verletzungen, nämlich das Unterbringen von Pflastern. Der erste Behälter des erfindungsgemäßen Verbandstoffbehälters ist daher zweckmäßigerweise als Pflasterspender ausgebildet. Bekannte Pflasterspender, die häufig in Gewerbebetrieben eingesetzt werden, werden mit Pflasterpackungen bestückt, die, wenn die Pflaster verbraucht sind, einfach ausgetauscht werden. Solche Pflasterpackungen, die auf dem Markt erhältlich sind, bestehen in der Regel aus Papier oder Pappe und weisen einzelne nach oben offene Taschen auf, in denen die Pflaster untergebracht sind und für eine einfache Entnahme nach oben herausragen. Bekannte Pflastereinsätze umfassen hintereinander mehrere Reihen von Pflastertaschen, die mit Pflastern bestückt sind. Am unteren Ende sind die mehreren Reihen häufig zusammengeheftet oder anderweitig eng miteinander verbunden, so daß der gesamte austauschbare Pflastereinsatz eine nach unten zusammenlaufende keilförmige Form besitzt. In einer besonders bevorzugten Ausführungsform des erfindungsgemäßen Verbandstoffbehälters ist der erste Behälter speziell für solche Pflastereinsätze der vorgenannten Art ausgelegt. Er ist daher dadurch gekennzeichnet, daß die Vorderwand und die Rückwand des ersten Behälters von der Öffnung zum Boden des Behälters keilförmig zusammenlaufen.

Der erfindungsgemäße Verbandstoffbehälter kann aus jedem geeigneten Material hergestellt sein, wobei er jedoch zweckmäßigerweise aus Kunststoff hergestellt ist. Dabei ist der Klappdeckel des ersten Behälters zweckmäßigerweise aus durchsichtigem Kunststoff hergestellt, was einen direkten Blick in das Innere des ersten Behälters und den noch vorhandenen Vorrat an darin befindlichem Verbandmaterial erlaubt.

Es ist weiterhin zweckmäßig, wenn in dem ersten Behälter und/oder dem zweiten Behälter des erfindungsgemäßen Verbandstoffbehälters Facheinteilungen und/oder Ablageeinrichtungen für das Unterbringen von Gegenständen vorgesehen sind. Im ersten Behälter können solche Einteilungen für das Unterbringen mehrerer gleicher oder verschiedener Verbandmaterial- bzw. Pflasterpackungen ausgelegt sein. Im zweiten Behälter können solche Einteilungen zweckmäßig für das Unterbringen bzw. das Auflegen von einem Meldeberichtsheft, Schreibwerkzeug und/oder einem Behälter mit Desinfektionslösung ausgelegt sein.

Damit der erfindungsgemäße Verbandstoffbehälter für eine Wandmontage geeignet ist, weist bei einer besonders bevorzugten Ausführungsform des Verbandstoffbehälters die Rückwand des zweiten Behälters Befestigungseinrichtungen, vorzugsweise eine oder mehrere durch die Rückwand hindurchtretende Öffnungen, besonders bevorzugt zwei durch die Rückwand hindurchtretende Öffnungen auf gleicher horizontaler Höhe für eine Befestigung des Verbandstoffbehälters an einer Wand oder einem Korpus auf.

Weitere Vorteile, Merkmale und Ausgestaltungsmöglichkeiten der vorliegenden Erfindung werden anhand der nachfolgenden Beschreibung zweier bevorzugter Ausführungsformen und der dazugehörigen Figuren erläutert.

Fig. 1a bis 5a zeigen verschiedene Ansichten und Zustände einer ersten Ausführungsform (a) und Fig. 1b bis 5b zeigen entsprechende Ansichten und Zustände einer zweiten Ausführungsform (b) des erfindungsgemäßen Verbandstoffbehälters.
- Fig. 1a und 1b: zeigen jeweils perspektivische schematische Darstellungen der Ausführungsformen (a) bzw. (b) des erfindungsgemäßen Verbandstoffbehälter schräg von oben.
- Fig. 2a, 3a, 4a und 2b, 3b, 4b: zeigen die erfindungsgemäßen Verbandstoffbehälter der Ausführungs formen (a) bzw. (b) gemäß Fig.1a bzw. 1b von der Seite in geschlossenem Zustand (Fig. 2a, 2b), mit geöffnetem Klappdeckel des ersten Behälters (Fig. 3a, 3b) bzw. mit hochgeklapptem ersten Behälter (Fig. 4a, 4b).
- Fig. 5a und 5b: zeigen jeweils Ansichten der Ausführungsformen (a) bzw. (b) der erfindungsgemäßen Verbandstoffbehälter gemäß Fig. 1a bzw. 1b von hinten.

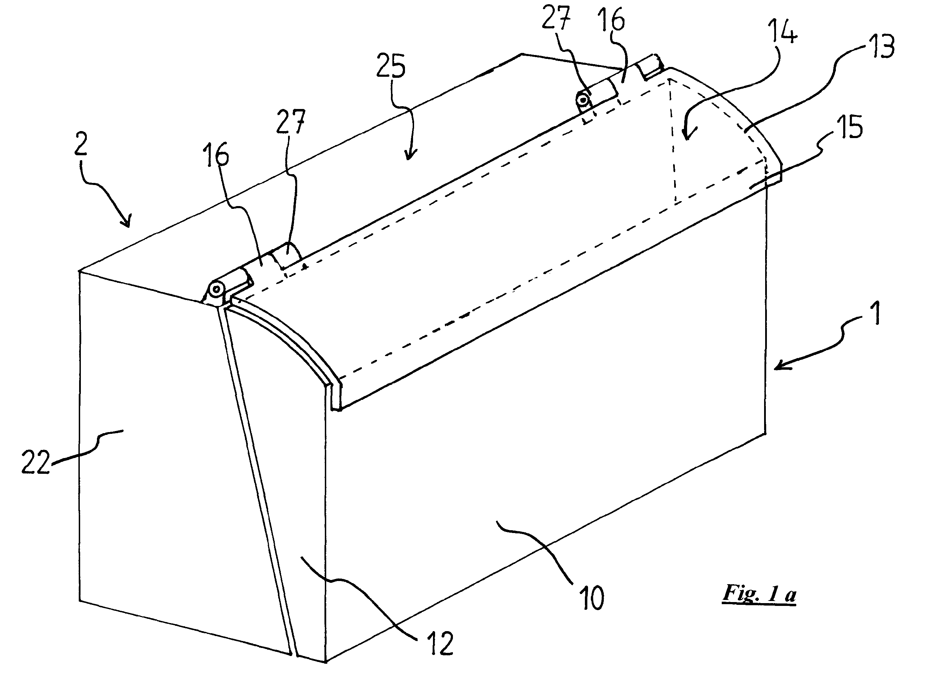

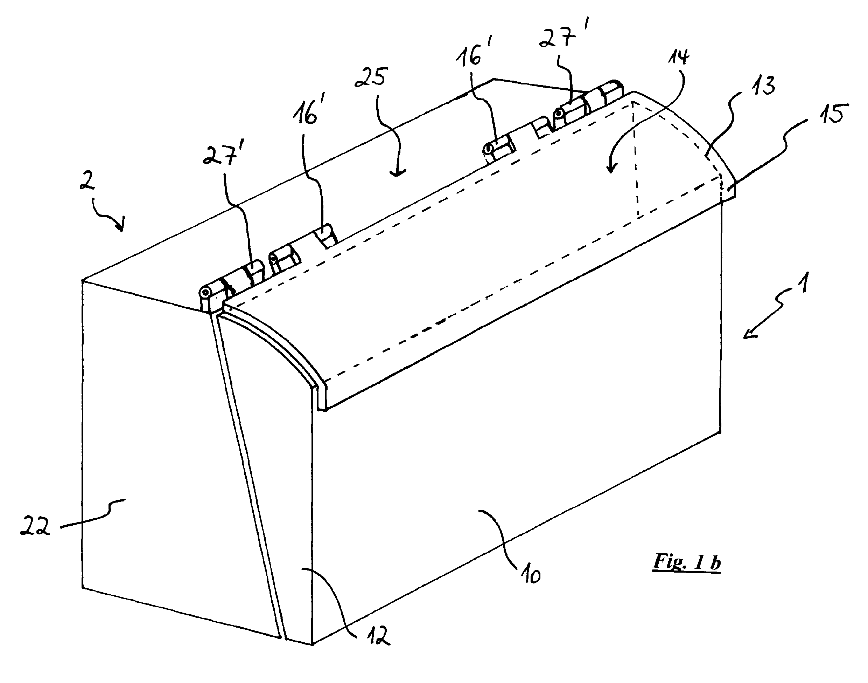

Die in den Fig. 1a bis 5a und 1b bis 5b dargestellten zwei Ausführungsformen des erfindungsgemäßen Verbandstoffbehälters umfassen jeweils einen ersten Behälter 1 mit einer Vorderwand 10, einer Rückwand 11, Seitenwänden 12 und 13 und einem Klappdeckel 15 zum Verschließen des ersten Behälters. Die Wände 10, 11, 12 und 13 definieren eine Öffnung 14, durch welche der erste Behälter 1 von oben zugänglich ist. Die zwei Ausführungsformen des erfindungsgemäßen Verbandstoffbehälters umfassen weiterhin einen zweiten Behälter 2 mit einer Rückwand 21, Seitenwänden 22 und 23, einer Oberwand 25 und einer Bodenwand 26. Die Wände 22, 23, 25 und 26 definieren eine gegenüber der Rückwand 21 in horizontaler Richtung nach vorne offene Behälteröffnung 24.

Bei der Ausführungsform gemäß den Fig. 1a bis 5a ist der Klappdeckel 15 an bzw. in der Nähe der in horizontaler Richtung vorderen Kante der Oberwand 25 des zweiten Behälters 2 über ein Scharnier 16 aufklappbar angelenkt. Bei der Ausführungsform gemäß den Fig. 1b bis 5b ist der Klappdeckel 15 an bzw. in der Nähe der oberen Kante der Rückwand 11 des ersten Behälters 1 über ein Scharnier 16' aufklappbar angelenkt. Im geschlossenen Zustand erstreckt sich der Klappdeckel 15 über die Öffnung 14 bis über die Kante der Vorderwand 10 des ersten Behälters hinaus.

Der erste Behälter 1 ist an bzw. in der Nähe der oberen Kante der Rückwand 11 des ersten Behälters 1 über ein Scharnier 27 (Fig. 1a bis 5a) bzw. Scharnier 27' (Fig. 1 b bis 5b) an bzw. in der Nähe der in horizontaler Richtung vorderen Kante der Oberwand 25 des zweiten Behälters klappbar angelenkt. Die Anordnung und Bemessung der Behälterwände ist so, daß die Rückwand 11 des ersten Behälters 1 die Behälteröffnung 24 des zweiten Behälters 2 im wesentlichen vollständig verschließt bzw. abdeckt, wenn sich der erste Behälter 1 im heruntergeklappten Zustand befindet.

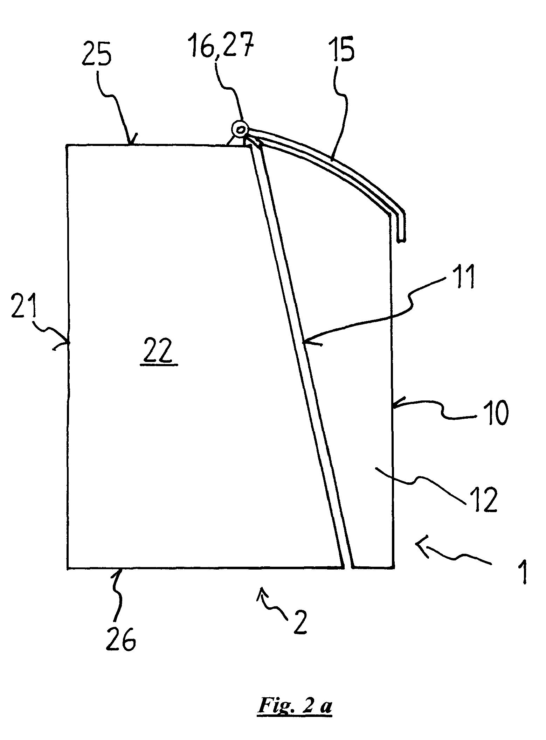

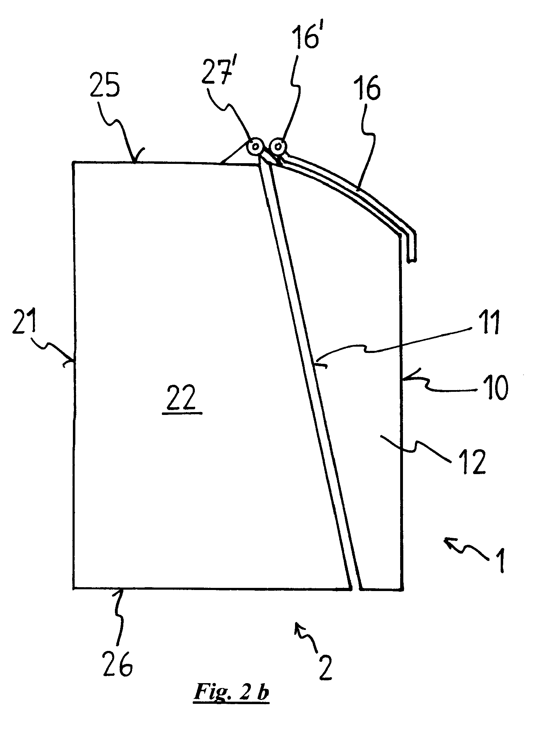

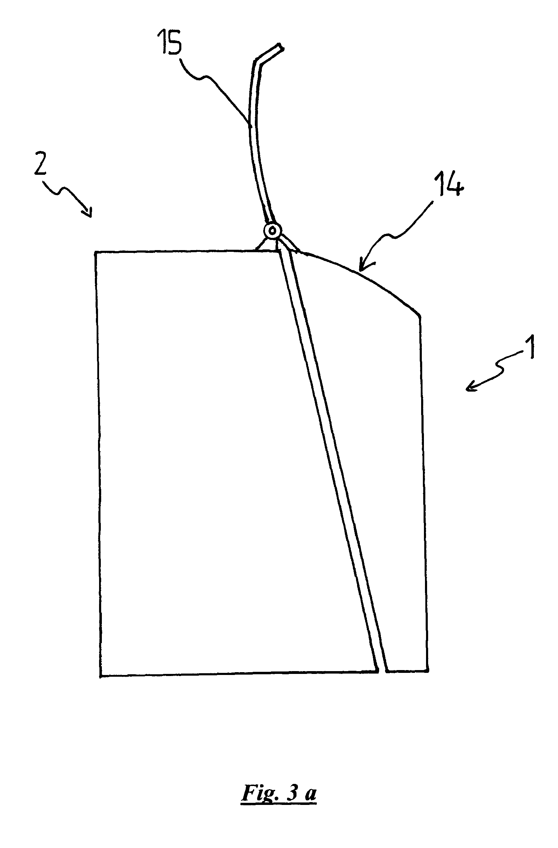

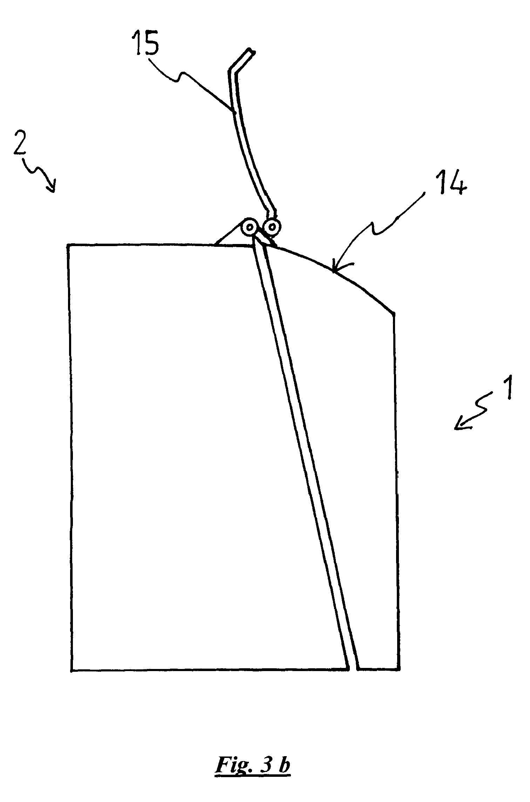

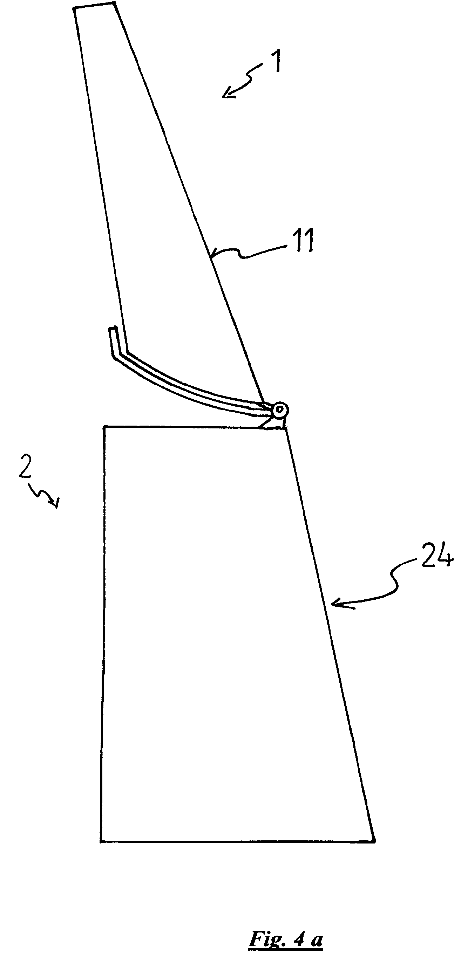

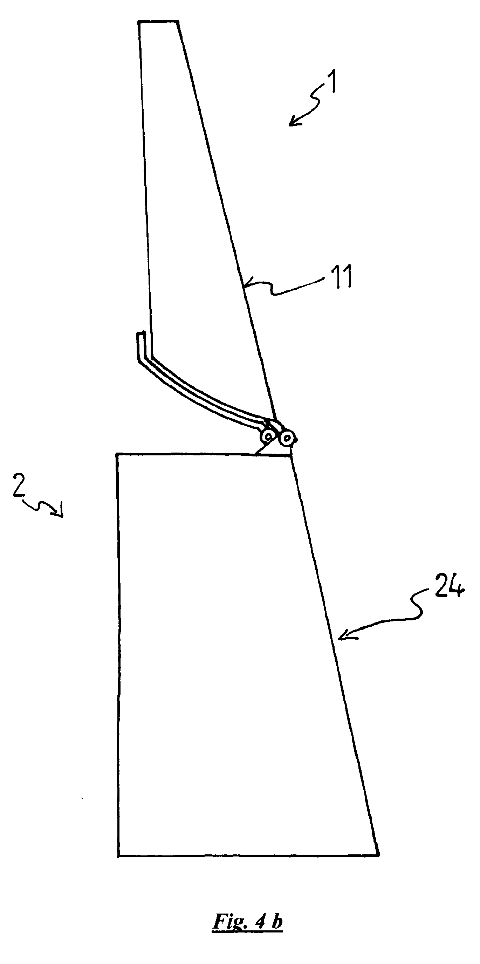

Die Fig. 2a bzw. 2b zeigen die zwei Ausführungsformen der erfindungsgemäßen Verbandstoffbehälter in geschlossenem Zustand, wie sie auch in Fig. 1a und 1b dargestellt sind. Fig. 3a bzw. 3b zeigen die gleichen Ansichten wie Fig. 2a bzw. 2b, jedoch mit jeweils geöffnetem Klappdeckel 15. In Fig. 4a bzw. 4b ist der erste Behälter 1 unter Freigabe der Behälteröffnung 24 des zweiten Behälters hochgeklappt. Dabei befindet sich die Rückwand 11 des ersten Behälters 1 hinter der Senkrechten auf das zweite Scharnier 27 bzw. 27', so daß der erste Behälter 1 in diesem hochgeklappten Zustand verbleibt und nicht gegen ein Herunterklappen gehalten werden muß. In Fig. 4a bzw. 4b ist auch zu erkennen, daß die Tiefe der Oberwand 25 des zweiten Behälters 2 so groß ist, daß sich die Abmessungen des ersten Behälters 1 im hochgeklappten Zustand nicht weiter als bis zu der durch die Rückwand 21 des zweiten Behälters 2 definierten vertikalen Ebene erstrecken.

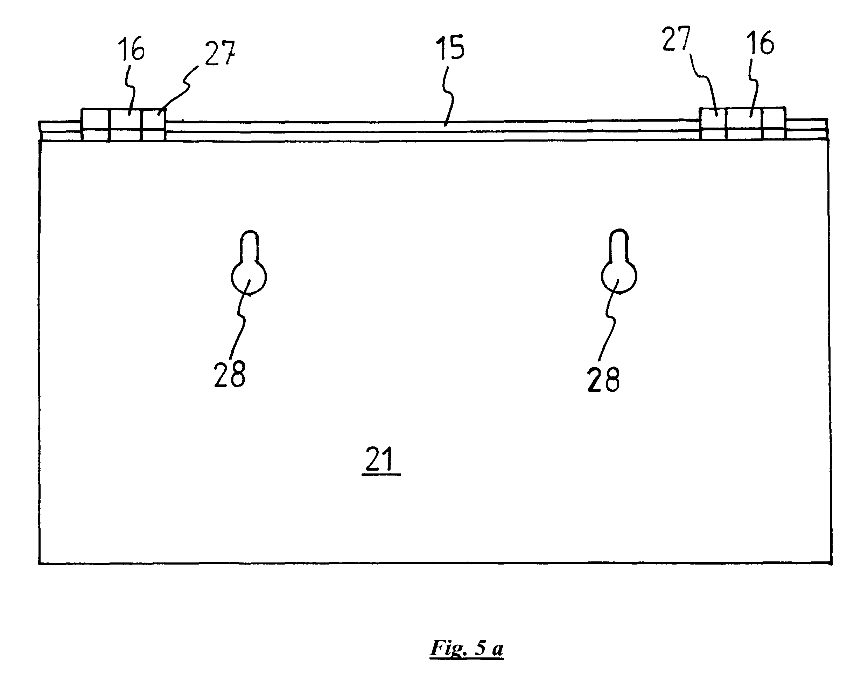

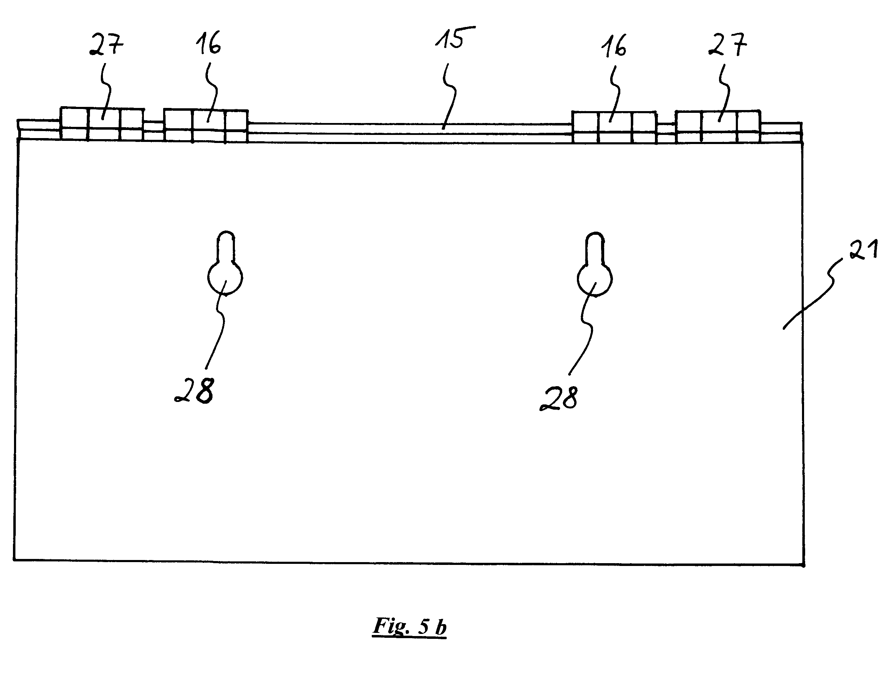

Fig. 5a bzw. 5b zeigen die zwei Ausführungsformen der erfindungsgemäßen Verbandstoffbehälter von hinten sowie die in der Rückwand 21 des zweiten Behälters 2 vorgesehenen Öffnungen 28 für eine Wandmontage.

## Patentansprüche

1. Verbandstoffbehälter mit einem ersten Behälter (1), vorzugsweise für Verbandmaterial, und einem zweiten Behälter (2), wobei
der erste Behälter (1) eine Vorderwand (10), eine Rückwand (11), Seitenwände (12, 13), eine in vertikaler Richtung nach oben offene Behälteröffnung (14) und einen Klappdekkel (15) zum Verschließen des Behälters (1) aufweist,
der zweite Behälter (2) eine Rückwand (21), Seitenwände (22, 23), eine Oberwand (25), eine Bodenwand (26) und eine gegenüber der Rückwand (21) in horizontaler Richtung nach vorne offene Behälteröffnung (24) aufweist,
der erste Behälter (1) an oder in der Nähe der oberen Kante der Rückwand (11) des ersten Behälters (1) über ein Scharnier (27, 27') an oder in der Nähe der in horizontaler Richtung vorderen Kante der Oberwand (25) des zweiten Behälters (2) klappbar angelenkt ist,
der Klappdeckel (15) an oder in der Nähe der in horizontaler Richtung vorderen Kante der Oberwand (25) des zweiten Behälters (2) oder an oder in der Nähe der oberen Kante der Rückwand (11) des ersten Behälters (1) über ein Scharnier (16, 16') aufklappbar angelenkt ist und sich im geschlossenen Zustand über die Öffnung (14) bis an die obere Kante der Vorderwand (10) oder über diese hinaus erstreckt,
die Anordnung und Bemessung der Behälterwände so ist, daß die Rückwand (11) des ersten Behälters (1) die Behälteröffnung (24) des zweiten Behälters (2) im wesentlichen vollständig verschließt bzw. abdeckt, wenn sich der erste Behälter im heruntergeklappten Zustand befindet.

2. Verbandstoffbehälter nach Anspruch 1, **dadurch gekennzeichnet, daß** die Tiefe der Oberwand (25) des zweiten Behälters (2) von der Vorderkante bis zur Hinterkante im Verhältnis zur Tiefe des gesamten ersten Behälters (1) wenigstens so groß ist, daß die Rückwand des ersten Behälters (1) im hochgeklappten Zustand in Richtung der Rückwand des zweiten Behälters (2) hinter der Senkrechten auf das Scharnier (27, 27') zu liegen kommt und sich die Abmessungen des ersten Behälters (1) dabei nicht weiter als bis zu der durch die Rückwand (21) des zweiten Behälters (2) definierten vertikalen Ebene erstrecken.

3. Verbandstoffbehälter nach einem der vorangegangenen Ansprüche, **dadurch gekennzeichnet, daß** der Klappdeckel (15) an oder in der Nähe der in horizontaler Richtung vorderen Kante der Oberwand (25) des zweiten Behälters (2) über ein Scharnier (16) aufklappbar angelenkt ist.

4. Verbandstoffbehälter nach Anspruch 3, **dadurch gekennzeichnet, daß** das Scharnier (16), über welches der Klappdeckel (15) an dem zweiten Behälter (2) angelenkt ist, und das Scharnier (27), über welches der erste Behälter (1) an dem zweiten Behälter (2) angelenkt ist, eine gemeinsame Scharnierachse aufweisen.

5. Verbandstoffbehälter nach einem der vorangegangenen Ansprüche, **dadurch gekennzeichnet, daß** die Vorderwand (10) und die Rückwand (11) des ersten Behälters (1) von der Öffnung (14) zum Boden des Behälters (1) keilförmig zusammenlaufen.

6. Verbandstoffbehälter nach einem der vorangegangenen Ansprüche, **dadurch gekennzeichnet, daß** der Verbandstoffbehälter aus Kunststoff hergestellt ist und/oder der Klappdeckel (15) aus durchsichtigem Kunststoff hergestellt ist.

7. Verbandstoffbehälter nach einem der vorangegangenen Ansprüche, **dadurch gekennzeichnet, daß** in dem ersten Behälter (1) und/oder dem zweiten Behälter (2) Facheinteilungen und/oder Ablageeinrichtungen für das Unterbringen von Gegenständen vorgesehen sind.

8. Verbandstoffbehälter nach einem der vorangegangenen Ansprüche, **dadurch gekennzeichnet, daß** die Rückwand (21) des zweiten Behälters (2) Befestigungseinrichtungen (28), vorzugsweise eine oder mehrere durch die Rückwand (21) hindurchtretende Öffnungen, besonders bevorzugt zwei durch die Rückwand (21) hindurchtretende Öffnungen auf gleicher horizontaler Höhe für eine Befestigung des Verbandstoffbehälters an einer Wand oder einem Korpus aufweist.
